# EUROPEAN PATENT APPLICATION

(11) **EP 4 394 381 A1**
(43) Date of publication of application: **03.07.2024**
(21) Application number: 22877857.7
(22) Date of filing: 19.09.2022
(51) Int. Cl.: G01N 33/52, G01N 35/00, G16Y 40/10

(54) **RAPID DRUG TEST METHOD, ONE-MINUTE RAPID DRUG TEST DEVICE, AND DISTRIBUTED DRUG TEST SYSTEM**

(30) Priority: 09.10.2021 CN 202111178153; 09.10.2021 CN 202111178163; 09.10.2021 CN 202111178172
(71) Applicant: Wuhan Nanodiagnosis for Health Biotechnology Co., Ltd., Wuhan, Hubei 430070 (CN)
(72) Inventor: JIN, Wei, Guangzhou, Guangdong 510000 (CN); ZHANG, Dejun, Guangzhou, Guangdong 510000 (CN); NIE, Liyan, Guangzhou, Guangdong 510000 (CN); WANG, Hailin, Guangzhou, Guangdong 510000 (CN)
(74) Representative: Cleanthous, Marinos
(86) International application number: PCT/CN2022/119591
(87) International publication number: WO 2023/056837

(57) **Abstract**

A rapid drug test method, a one-minute rapid drug test device, and a distributed drug test system are provided. The rapid drug test method includes: providing drug abuse model, wherein the drug abuse model is used for determining, according to the visual effect change of test paper, whether the drug content of a sample to be tested exceeds critical value; enabling the sample to be tested to react with the test paper; before the test paper fully reacts, obtaining the actually measured visual effect change of the test paper; and preliminarily determining, in combination with the actually measured visual effect change and the drug abuse model, whether the drug content of the sample to be tested exceeds the critical value. The rapid drug test method, the one-minute rapid drug test device, and the distributed drug test system provided by the present disclosure can effectively shorten the test time, improve the test efficiency, and further reduce the difficulty of the general test.

## Description

### CROSS-REFERENCE TO RELATED APPLICATION

This application claims priorities to the following Chinese Patent Applications, the contents of which are incorporated herein by reference in its entirety.

| Serial number | Application date | Application number | Name |
|---|---|---|---|
| 1 | 10/9/2021 | 202111178153.1 | RAPID DRUG TEST METHOD, ONE-MINUTE RAPID DRUG TEST DEVICE, AND DISTRIBUTED DRUG TEST SYSTEM |
| 2 | 10/9/2021 | 202111178163.5 | ONE-MINUTE RAPID DRUG TEST DEVICE |
| 3 | 10/9/2021 | 202111178172.4 | DISTRIBUTED DRUG TEST SYSTEM |

### TECHNICAL FIELD

The present disclosure relates to the technical field of drug test, and in particular to a rapid drug test method, a one-minute rapid drug test device, and a distributed drug test system.

### BACKGROUND

Drug abuse test devices can be divided into rapid drug test device and precision test device. Among them, the rapid drug test device generally carries out a test through test paper. Although the rapid drug test device has low test precision, it has a small size and high test speed, and is easy to carry, such as a drug test rod, etc. The precision test device is generally provided with larger equipment such as a centrifuge or an element tester. Although the precision test device has high test precision, it is bulky and heavy and is difficult to carry for operation, such as a urine test device, a blood test device, a hair test device, a sweat test device, a saliva test device, and a kidney function test device, etc.

During inspections at specific locations, supervisors generally need to conduct drug abuse tests on all individuals (general test). Extremely long test time can easily cause confusion and greatly increase the difficulty of general testing. Therefore, at the present, the supervisors generally use the rapid drug test device to conduct the general testing on the testees.

Taking the drug test rod as an example, the current test process of using the rapid drug test device is as follows:
① A testee puts the drug test rod into their mouth, allwing saliva, serving as the sample to be tested, to infiltrate the test paper through a hole in the drug test rod and then reacts with the test paper.
② If the testee has taken drugs, the test paper will gradually change the color or produce a fluorescent visual effect change.
③ After about 3 min, the test paper can fully react with the saliva. At this time, the supervisor can check the visual effect change of the test paper to determine whether the testee has taken drugs.

It generally takes 5 min or longer for current drug test devices to complete a drug abuse test. Although the test speed is higher than that of the precision test device, if a large number of persons need to be tested for general testing, it is still difficult for the test speed to meet the requirements.

Therefore, the existing drug abuse test method needs to be improved to further shorten the test time and improve the test efficiency.

The above information disclosed in the background section is only intended to enhance the understanding of the background of the content of the present disclosure, and may therefore include information that does not constitute the prior art that is currently known to those skilled in the art.

### SUMMARY

One objective of the present disclosure is to provide a rapid drug test method, a one-minute rapid drug test device, and a distributed drug test system, which can effectively shorten the test time, improve the test efficiency, and further reduce the difficulty of the general test.

In order to achieve the above objectives, in one aspect, the present disclosure provides a rapid drug test method, including:
providing a drug abuse model, wherein the drug abuse model is used for determining, according to a visual effect change of test paper, whether the drug content of a sample to be tested exceeds critical value;
enabling the sample to be tested to react with the test paper;
before the test paper fully reacts, obtaining an actually measured visual effect change of the test paper;
and preliminarily determining, in combination with the actually measured visual effect change and the drug abuse model, whether the drug content of the sample to be tested exceeds the critical value.

Optionally, the determining, according to a visual effect change of test paper, whether the drug content of a sample to be tested exceeds the critical value includes: preparing a calibration sample with the drug content equal to the critical value; enabling the calibration sample to react with calibration test paper, obtaining visual effect intensities of the calibration test paper at several moments in the whole reaction process, and calculating visual effect intensity change rates corresponding to the various visual effect intensities; and drawing a drug abuse critical curve that represents a mapping relationship between the visual effect intensity change rates and the visual effect intensities.

Optionally, the obtaining an actually measured visual effect change of the test paper includes: obtaining visual effect intensities of the test paper at several moments within a specified time period before the sample to be tested fully reacts with the test paper, and calculating visual effect intensity change rates corresponding to the various visual effect intensities; and drawing an actually measured curve that represents a mapping relationship between the visual effect intensity change rates of the test paper and the visual effect intensities.

Optionally, the start point of the specified time period is the moment when the test paper starts to react.

Optionally, the preliminarily determining whether the drug content of the sample to be tested exceeds the critical value specifically includes: preliminarily determining, according to the relative position relationship between the drug abuse critical curve and the actually measured curve, whether the drug content of the sample to be tested exceeds the critical value.

Optionally, after the preliminary determination result is obtained, the method further includes: carrying out a retest with higher test precision on the testees, who are preliminarily determined that the drug content of the sample to be tested exceeds the critical value.

Optionally, after the carrying out the retest with higher test precision on the testees, who are preliminarily determined that the drug content of the sample to be tested exceeds the critical value, the method further includes: obtaining a past drug abuse records of the testees.

Optionally, after the obtaining of the past drug abuse records of the testees, the method further includes: updating the drug abuse record.

In another aspect, a one-minute rapid drug test device is provided, including:
a plug-in drug test card, configured to enable a sample to be tested to react with test paper; and
a portable terminal, configured to:
provide a drug abuse model, wherein the drug abuse model is used for determining, according to a visual effect change of test paper, whether the drug content of a sample to be tested exceeds critical value;
before the test paper fully reacts, obtain an actually measured visual effect change of the test paper; and
preliminarily determine, in combination with the actually measured visual effect change and the drug abuse model, whether the drug content of the sample to be tested exceeds the critical value.

In still another aspect, a distributed drug test system is provided, including a central device and several one-minute rapid drug test devices described above.

The central device is provided with precision test device; the one-minute rapid drug test device and the precision test device are both configured to conduct a drug abuse test; a test speed of the one-minute rapid drug test device is higher than a test speed of the precision test device; and the test precision of the precision test device is higher than the test precision of the one-minute rapid drug test device.

The present disclosure has the beneficial effects that the rapid drug test method, the one-minute rapid drug test device, and the distributed drug test system are provided. After the drug abuse model is provided, the visual effect change of the test paper can be obtained early, so that the test time is shortened, and the test efficiency is improved.

### BRIEF DESCRIPTION OF THE DRAWINGS

To describe the technical solutions in the embodiments of the present disclosure or in the related art more clearly, the following briefly introduces the accompanying drawings for describing the embodiments or the related art. Apparently, the accompanying drawings in the following description show merely some embodiments of the present disclosure, and a person of ordinary skill in the art may still derive other drawings from the accompanying drawings without creative efforts.
FIG. 1 is a flowchart of a rapid drug test method provided according to an embodiment;
FIG. 2 is a schematic diagram showing that an actually measured curve falls into a positive region provided according to an embodiment;
FIG. 3 is a schematic diagram of a top view of a distributed drug test system provided according to an embodiment;
FIG. 4 is a schematic cross-sectional diagram of a plug-in drug test card provided according to an embodiment;
FIG. 5 is a schematic diagram of a top view of a lower shell provided according to an embodiment;
FIG. 6 is a schematic diagram of insertion of a sampler and a shell provided according to an embodiment;
FIG. 7 is a schematic diagram of a one-minute rapid drug test device provided according to an embodiment; and
FIG. 8 is a structural block diagram of a distributed drug test system provided according to an embodiment.

### In the drawings:

100: plug-in drug test card; 200: portable terminal; 300: central device;
1: shell; 101: upper shell; 1011: observation window; 102: lower shell; 1021: test paper slot; 1022: drainage baffle plate; 103: test chamber; 104: shell insertion slot; 105: first limiting boss; 106: extrusion boss; 107: inclined channel; 108: second limiting boss; 1081: plate passing notch; 109: buffer space;
2: sampler; 201: fiber bundle; 202: handheld part; 203: clamping plate;
3: test paper;
4: information acquisition unit;
5: data processing unit;
6: terminal side output device;
7: terminal insertion slot;
8: central device insertion slot;
9: power supply system;
10: information inputting module;
11: first memory;
12: data search module;
13: precision test device;
14: second memory;
15: update module;
16: drug abuse critical curve;
17: positive region;
18: negative region; and
19: actually measured curve.

### DETAILED DESCRIPTION OF THE EMBODIMENTS

In order to make the objectives, features, and advantages of the present disclosure more obvious and understandable, the technical solutions in the embodiments of the present disclosure will be clearly and completely described below with reference to the drawings in the embodiments of the present disclosure, and it is obvious that the embodiments described below are only a part of the embodiments of the present disclosure but not all of them. All other embodiments obtained by a person of ordinary skill in the art based on the embodiments of present disclosure without making creative efforts shall fall within the protection scope of present disclosure.

In the descriptions of the present disclosure, it should be understood that when an assembly is considered to be "connected" to another assembly, the assembly can be directly connected to the another assembly or there may be an intermediate assembly. When an assembly is considered to be "arranged" on another assembly, the assembly can be directly arranged on the another assembly or there may be an intermediate assembly.

In addition, orientations or positional relationships indicated by the terms "long", "short", "inside", "outside", and the like are orientations or positional relationships as shown in the drawings, and are only for the purpose of facilitating the description of the present disclosure instead of indicating or implying that devices or elements indicated must have particular orientations, and be constructed and operated in the particular orientations, so that these terms are not construed as limiting the present disclosure.

The present disclosure will be described in detail below in combination with specific implementations shown in the accompanying drawings. However, these implementations do not limit the present disclosure, and any transformations made by those of ordinary skill in the art to structures, methods, or functions according to these implementations fall within the protection scope of the present disclosure.

### Embodiment I

This embodiment provides a rapid drug test method, which is applicable to an application scenario where supervisors conduct drug abuse test on testees. First, a visual effect change of test paper can be obtained early, thus shortening the test time. Then, the testee who is preliminarily determined as having taken drugs is retested, so as to lower the risk of misjudgment.

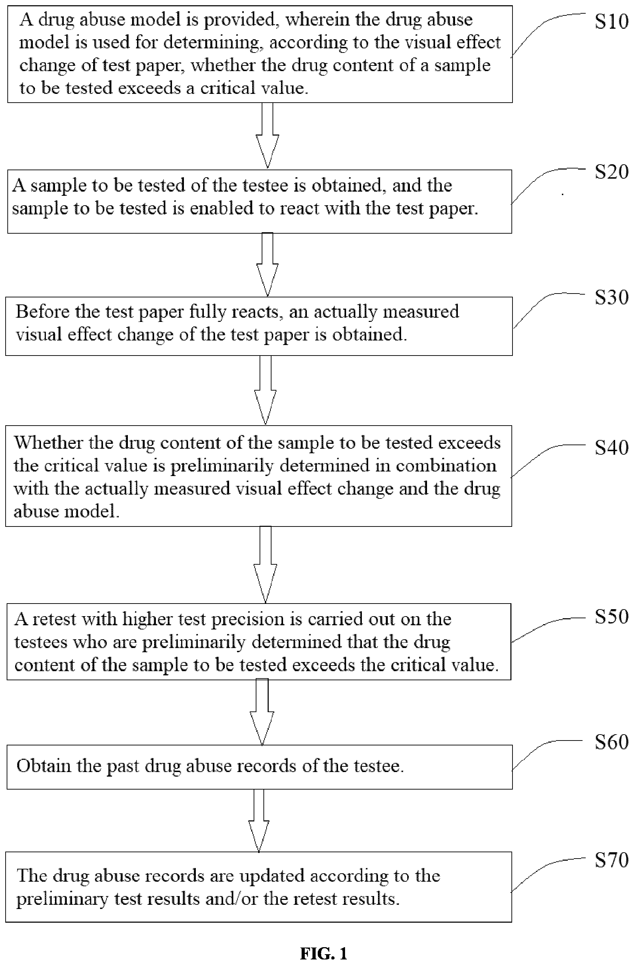

Referring to FIG. 1, the rapid drug test method of this embodiment includes step S10 to step S70 below.

S10: A drug abuse model is provided, wherein the drug abuse model is used for determining, according to the visual effect change of test paper, whether the drug content of a sample to be tested exceeds a critical value.

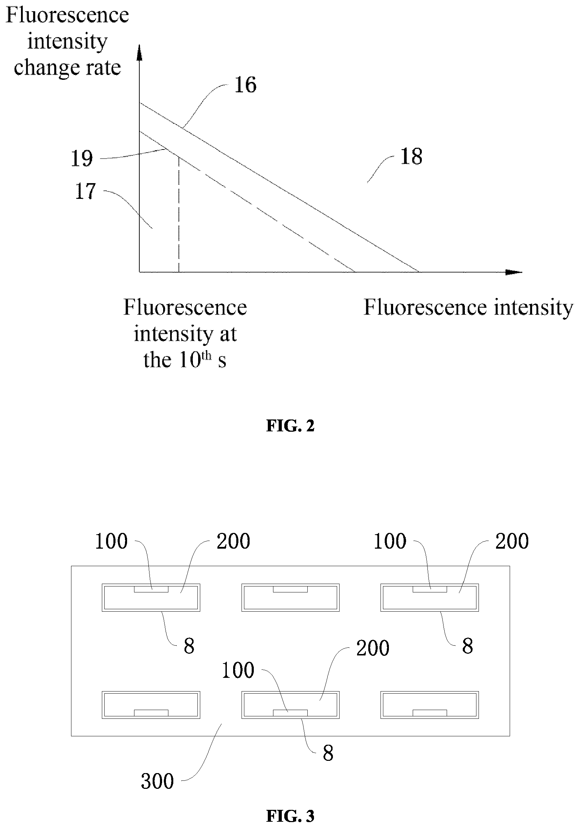

Referring to FIG. 2, in this embodiment, the drug abuse model can be obtained using step S101 to step S104:
S101: critical value of the drug content specified by the relevant laws or documents is found, wherein if the drug content of the sample is greater than the critical value, it is considered that the testee has taken drugs and if the drug content of the sample is less than the critical value, it is considered that the testee has not taken drugs.
S102: A calibration sample having a drug content equal to the critical value is prepared.
S103: The calibration sample is enabled to react with calibration test paper; visual effect intensities of the calibration test paper at several moments in the whole reaction process are obtained; and visual effect intensity change rates corresponding to the various visual effect intensities are calculated.
S104: A drug abuse critical curve 16 that represents a mapping relationship between the visual effect intensity change rates of the calibration test paper and the visual effect intensities is drawn.

Generally, as the reaction time increases, the visual effect intensity (a fluorescence intensity or a color depth) of the calibration test paper will change after the calibration test paper reacts with drugs, and the change rate will also change over time.

For example, the calibration test paper is fluorescent test paper that is used in competitive immunoassay. After the reaction between the calibration test paper and the calibration sample starts, the visual effect intensity change rate of the calibration test paper at the beginning is large. For example, the fluorescence intensity of the calibration test paper rapidly increases from a smaller value to a larger value. That is, the change rate of the fluorescence intensity is relatively large. As the reaction time increases, the visual effect intensity change rate of the calibration test paper gradually decreases. That is, although the fluorescence intensity of the calibration test paper is increasingly high, the rising rate of the fluorescence brightness increases is increasingly low. Finally, when the fluorescence intensity of the calibration test paper remains basically unchanged, that is, when the rising rate of the fluorescence brightness is almost zero, it can be considered that the calibration test paper has fully reacted with the calibration sample. Generally, it takes about 3 min from the beginning of the reaction of the calibration test paper to the full reaction.

When the reaction between the calibration sample and the calibration test paper starts, the fluorescence intensity of the calibration test paper can be tested every 0.1 s to 0.3 s until the calibration sample and the calibration test paper fully react with each other. At this time, the fluorescence intensities corresponding to various moments after the reaction starts can be obtained. The fluorescence intensity change rates corresponding to the various moments can be calculated according to the various fluorescence intensities. Then, a series of discrete points can be drawn on a rectangular coordinate system by taking the fluorescence intensity as an x-coordinate (X-axis) and taking the falling rate of the fluorescence intensity as a y-coordinate (Y-axis), and a fitted curve is calculated according to the various discrete points. The fitted curve is denoted as a drug abuse critical curve 16. It can be understood that the drug abuse critical curve 16 is used to represent, when the drug content of the calibration sample is the critical value, that the visual effect change rate (the fluorescence intensity change rate) of the calibration test paper changes as the visual effect intensity (the fluorescence intensity) changes.

It should be noted that regions on and below the drug abuse critical curve 16 are both positive regions 17, and a region above the drug abuse critical curve 16 is a negative region 18. In some other embodiments, if the calibration test paper is the fluorescent test paper used in general immunochromatography, regions on and above the drug abuse critical curve 16 are both positive regions 17, and a region below the drug abuse critical curve 16 is a negative region 18.

Optionally, the precision of the drug abuse critical curve 16 can be further optimized through a method such as averaging in multiple experiments.

S20: A sample to be tested of the testee is obtained, and the sample to be tested is enabled to react with the test paper.

It should be noted that the functions and components of the calibration test paper are the same as those of the test papers are the same, but they have different names for the purpose of distinguishing in description for better understanding.

In the present disclosure, the sample to be tested may be saliva or blood of the testee, and this embodiment does not limit this.

S30: Before the test paper fully reacts, an actually measured visual effect change of the test paper is obtained.

Optionally, the actually measured visual effect change of the test paper is obtained using step S301 and step S302:
S301: Visual effect intensities of the test paper at several moments within a specified time period before the sample to be tested fully reacts with the test paper are obtained, and visual effect intensity change rates corresponding to the various visual effect intensities are calculated.
S302: An actually measured curve 19 that represents a mapping relationship between the visual effect intensity change rates of the test paper and the visual effect intensities is drawn.

In the traditional drug test method, visual effect parameters are usually obtained and compared after the test paper fully reacts, so that the test time is at least 5 min. An important difference between the rapid drug test method provided in this embodiment and the traditional drug test method is that the visual effect parameters are obtained and compared before the test paper fully reacts. Generally, it only takes less than 60 seconds to obtain a determining result, which greatly shortens the test time and improves the test efficiency.

For example, the test paper is fluorescent test paper that is used in competitive immunoassay. Step S30 may include following detailed operations:

Within a specified time period after the reaction between the test paper and the sample to be tested starts, for example, within the previous 10 s after the reaction starts, the fluorescence intensity of the test paper is tested every 0.5 s to 2 s, thus obtaining the fluorescence intensities corresponding to various moments within the specified time period. The fluorescence intensity change rates corresponding to the various moments are calculated according to the various fluorescence intensities. Then, a series of discrete points can be drawn on a rectangular coordinate system by taking the fluorescence intensity as an x-coordinate (X-axis) and taking the falling rate of the fluorescence intensity as a y-coordinate (Y-axis), and a fitted curve is calculated according to the various discrete points. The fitted curve is denoted as an actually measured curve 19. It should be noted that different drug contents require different specified time periods. If the drug content is large, data acquisition and determination can generally be completed in the precious 10 s after the reaction starts. If the drug content is smaller, it is necessary to prolong the specified time period to the previous 50 s after the reaction starts. In summary, the data acquisition and determination can be completed within 50 s after the reaction starts.

It can be understood that the actually measured curve 19 is used to represent, when the sample to be tested is tested, that the visual effect change rate (the fluorescence intensity change rate) of the test paper changes as the visual effect intensity (the fluorescence intensity) changes.

S40: Whether the drug content of the sample to be tested exceeds the critical value is preliminarily determined in combination with the actually measured visual effect change and the drug abuse model.

It can be understood that whether the drug content of the sample to be tested exceeds the critical value can be preliminarily determined according to a relative position relationship between the drug abuse critical curve 16 and the actually measured curve 19. Specifically, if the actually measured curve 19 falls into the positive regions 17, it is preliminarily determined that the drug content exceeds the critical value, that is, the testee has taken drugs. If the actually measured curve 19 falls into the negative region 18, it is preliminarily determined that the drug content does not exceed the critical value, that is, the testee has not taken drugs.

S50: A retest with higher test precision is carried out on the testees who are preliminarily determined that the drug content of the sample to be tested exceeds the critical value.

It can be understood that a main test tool used for the preliminary test in the above step is the test paper. Although the test paper has high test speed, the precision of the test paper is lower than some precision test devices such as the urine test device, the blood test device, the hair test device, the sweat test device, the saliva test device, or the kidney function test device. Therefore, in order to minimize the risk of misjudgment, a retest operation can be added.

S60:Obtain the past drug abuse records of the testee.

Generally, test results of all drug abuse tests carried out by a supervision department will be recorded, and drug abuse records that contain drug abuse histories of all testees that had been tested are formed. Generally, the drug abuse record can include information such as the name, the ID card number, the age, the gender, the appearance, the fingerprints, the height, the weight, the native place, time when drug abuse was first found, and the number of times when drug abuse was found. If a testee has no history of drug abuse, the drug abuse record of the testee is empty.

Obtain the past drug abuse records of the testee. If it is found that the testee has a history of drug abuse, a sharp lookout should be kept on the testee.

S70: The drug abuse records are updated according to the preliminary test results and/or the retest results.

The drug abuse records are continuously updated to avoid information lag or information interruption.

The rapid drug test method provided in this embodiment obtains the actually measured visual effect changes of the test paper before the test paper fully reflects, and makes a preliminary judgment on whether the drug content exceeds the standard. This can greatly shorten the test time during the preliminary test, and even complete the test within 1 min, which has significant promotional value.

### Embodiment II

This embodiment provides a distributed drug test system, which can be applied to the rapid drug test method provided in Embodiment I. The distributed drug test system and the rapid drug test method have the same functions and beneficial effects.

The distributed drug test system is applicable to an application scenario where the supervisors carry out drug abuse tests on the testees. Firstly, a one-minute rapid drug test device is used to test the testees. The one-minute rapid drug test device makes a judgment early, thus shortening the test time. If the test result of the one-minute rapid drug test device indicates that the testee has taken drugs, the testee will be brought to the central device for an retest with higher precision, thus lowering the risk of misjudgment.

Referring to FIG. 3, the distributed drug test system provided in this embodiment includes a central device 300 and several one-minute rapid drug test devices. The central device 300 is provided with several central device insertion slot 8, and each of the central device insertion slots 8 is used for inserting one of the one-minute rapid drug test devices.

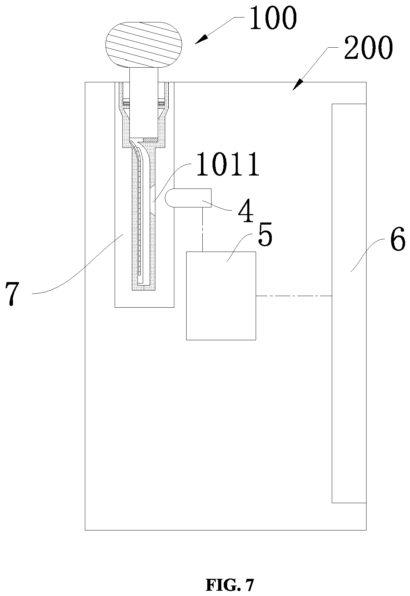

Referring to FIG. 7, each one-minute rapid drug test device provided in this embodiment includes plug-in drug test card 100 and portable terminal 200.

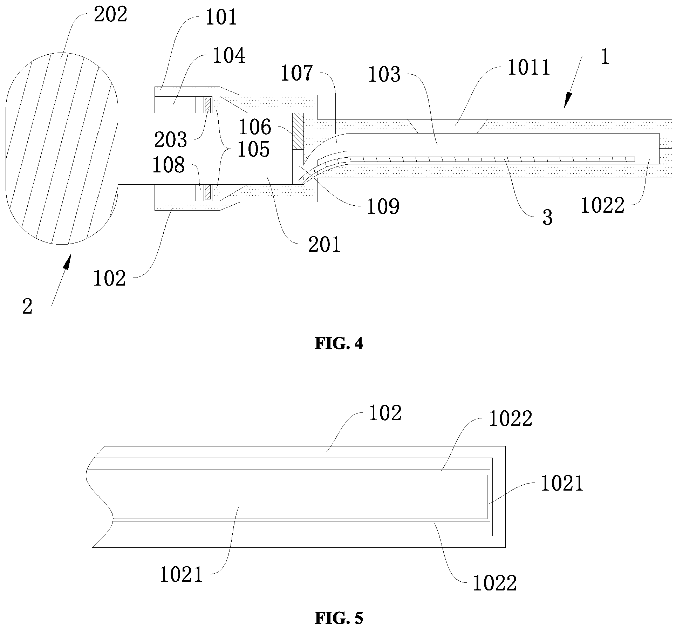

As shown in FIG. 4, the plug-in drug test card 100 includes a sampler 2 configured to obtain a sample to be tested, a shell 1 connected to the sampler 2 in a plugging manner, and test paper 3 located in the shell 1. The test paper 3 is configured to react with the sample to be tested. The portable terminal 200 is detachably connected to the plug-in drug test card 100 and is configured to obtain a visual change of the test paper 3 and generate a test result according to the visual change.

Further, a test chamber 103 for placing the test paper 3 is arranged inside the shell 1, and a shell insertion slot 104 communicated with the test chamber 103 through an inclined channel 107 is arranged on an end surface of one end of the shell 1. Optionally, the shell 1 includes an upper shell 101 and a lower shell 102 connected to the upper shell by a buckle. After the upper shell 101 and the lower shell 102 are buckled together, the test chamber 103, the inclined channel 107, and the shell insertion slot 104 can be formed inside. Further, the upper shell 101 is provided with an observation window 1011.

The test paper 3 is located in the test chamber 103 to react with the sample to be tested. For example, the test paper 3 can be reaction test paper containing fluorescent nanoparticles or a colloidal gold test paper 3. After reacting with the sample to be tested with an excessive drug content, the test paper 3 will have a visual effect change, such as, generating fluorescence or changing in color. The visual effect change of the test paper 3 can be observed through the observation window 1011. Drugs can be opium, heroin, methamphetamine, morphine, marijuana, cocaine, and other narcotic drugs and psychoactive drugs which are regulated by the state and can make people addictive.

The sampler 2 includes a fiber bundle 201 connected to the shell insertion slot 104 in a plugging manner, and a handheld part 202 fixed at one end of the fiber bundle 201 away from the shell 1. A surface of the handheld part 202 has an anti-skid boss. Further, the anti-skid boss is a dot protruding out of the surface of the handheld part 202.

A terminal insertion slot 7 is arranged on one surface of the portable terminal 200, and the plug-in drug test card 100 is connected to the terminal insertion slot 7 in a plugging manner. An information acquisition unit 4 configured to obtain the visual effect change is arranged at a position, corresponding to the observation window 1011, in the terminal insertion slot 7. Optionally, the information acquisition unit 4 is a fluorescence sensor or an image sensor. Further, the portable terminal 200 is further provided with a data processing unit 5 and a terminal side output device 6. The data processing unit 5 is connected to the information acquisition unit 4 and is configured to process data acquired by the information acquisition unit 4 and generate the test result. The terminal side output device 6 is electrically connected to the data processing unit 5 and is configured to provide displaying of at least one of pictures, characters, videos, sounds, or light for a supervisor according to the test result. Correspondingly, the terminal side output device 6 may include at least one of a display screen, a buzzer, or an indicator lamp.

For example, the terminal side output device 6 may include a display screen:
When the terminal side output device 6 is a display screen and the one-minute rapid drug test device obtains a positive result, the display screen outputs word "Positive".

For example, the terminal side output device 6 may include an indicator lamp:
When the terminal side output device 6 is a bulb and the one-minute rapid drug test device obtains a positive result, a red lamp is on, and a green lamp is off.

When the terminal side output device 6 is a bulb and the one-minute rapid drug test device obtains a negative result, the red lamp is off, and the green lamp is on.

Preferably, the terminal side output device 6 can also be connected to an alarm system through radio, a network, WIFI, and Bluetooth. When the result is positive, the terminal side output device 6 activates the alarm system to produce an alarm, so that more working personnel can arrive at the scene in a timely manner to prevent the person with the positive result from escaping.

Optionally, the size of the portable terminal 200 is similar to that of a mobile phone, making it easy to carry.

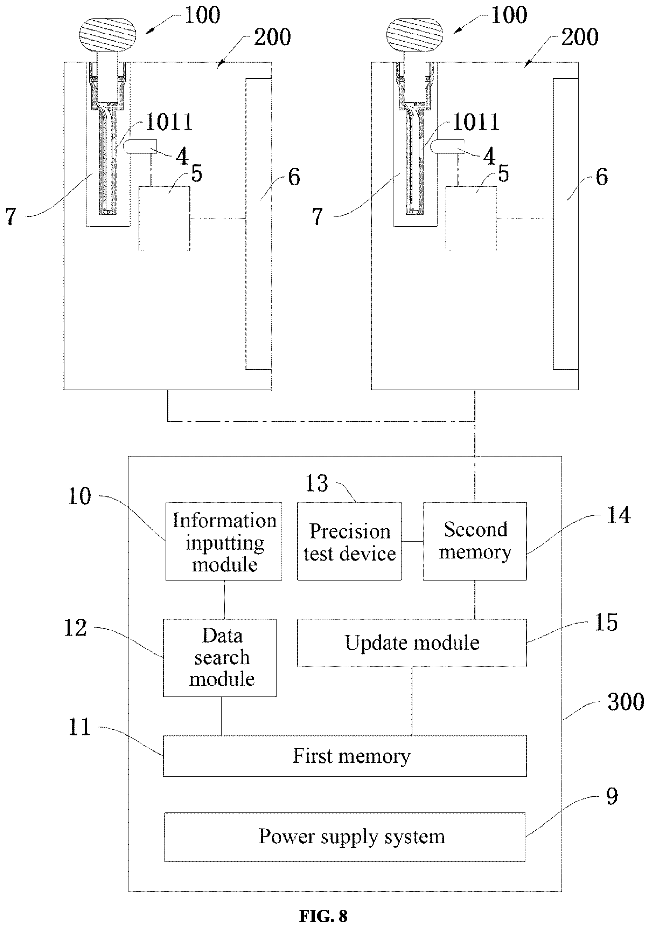

Referring to FIG. 8, the central device 300 is provided with a power supply system 9, an information inputting module 10, a first memory 11, a data search module 12, a precision test device 13, a second memory 14, and an update module 15. The power supply system 9 is configured to supply power to the information inputting module 10, the first memory 11, the data search module 12, the precision test device 13, the second memory 14, and the update module 15.

The precision test device 13 is configured to conduct a drug abuse test. Further, a test speed of the one-minute rapid drug test device is higher than a test speed of the precision test device 13; and the test precision of the precision test device 13 is higher than the test precision of the one-minute rapid drug test device. The precision test device 13 can include at least one of a urine test device, a blood test device, a hair test device, a sweat test device, a saliva test device, or a kidney function test device.

The information inputting module 10 is configured to obtain identity information of testees. Optionally, the identity information can be an ID card number, fingerprint information, facial information, or the like. Correspondingly, the information inputting module 10 can be an entry device configured to input the ID card number, a fingerprint module configured to input fingerprints, a camera configured for face recognition, or the like. Further, the entry device can be a touchable screen, a keyboard, or the like.

The first memory 11 is configured to store all drug abuse records that have been checked by a supervision department. Optionally, the drug abuse record can include the name, the ID card number, the age, the gender, the appearance, the fingerprints, the height, the weight, the native place, time when drug abuse was first found, and the number of times when drug abuse was found.

The data search module 12 is electrically connected to the information inputting module 10 and the first memory 11 respectively and is configured to call, according to the identity information, the drug abuse records related to the testee.

The second memory 14 is electrically connected to the information inputting module 10 and is configured to record the identity information of the testee and the test result of the precision test device 13.

The update module 15 is electrically connected to the first memory 11 and the second memory 14 respectively and is configured to update the identity information of the testee and the test result of the precision test device 13 in the second memory 14 to the first memory 11.

Optionally, a connection interface is arranged at a bottom of the portable terminal 200; a connection terminal is arranged at a slot bottom of each of the central device insertion slots 8. When the portable terminal 200 is inserted into the central device insertion slot 8, the connection terminal is plugged into the connection interface and achieves electrical connection, thereby charging the portable terminal 200 and achieving information transmission between the portable terminal 200 and the central device 300. Of course, the portable terminal 200 and the central device 300 can also achieve information transmission through wireless communications.

It should be noted that the drug abuse model mentioned in step S10 of Embodiment I can be stored in advance in the data processing unit 5 of the portable terminal 200 for calling at any time. The plug-in drug test card 100 provided in this embodiment can be configured to perform step S20 in Embodiment I. The portable terminal 200 provided in this embodiment can be configured to perform step S30 and step S40 in Embodiment I. The precision test device 13 provided in this embodiment can be configured to perform step S50 in Embodiment I. The central device 300 provided in this embodiment can be configured to perform step S60 and step S70 in Embodiment I.

The working process of the distributed drug test system provided in this embodiment is as follows:
① During monitoring, the distributed drug test system is first transported to a base camp. Some supervisors, as retest personnel, are sent to the base camp for stationing and guarding the central device 300. Other supervisors, as general test personnel, each carry a one-minute rapid drug test device to carry out a general test on testees in the crowd.
② When using the one-minute rapid drug test device for testing, the general test personnel need to pull out the plug-in drug test card 100 from the terminal insertion slot 7 and then put the portable terminal 200 into the pocket. Afterwards, the general test personnel hold the shell 1 in one hand and pinch the handheld part 202 of the sampler 2 with the other hand to pull the sampler 2 out of the shell insertion slot 104, and then place the other end of the fiber bundle 201 into the mouth of the testee. Due to the excellent water absorption capacity of the fiber bundle 201, the fiber bundle can be completely infiltrated by the saliva of the testee in a few seconds. That is, the fiber bundle 201 is covered with the saliva of the testee. The testee opens the mouth, and the supervisor takes out the fiber bundle 201, thus completing the obtaining of the sample to be tested.
② The general test personnel insert the fiber bundle 201 into the shell insertion slot 104, and then insert the plug-in drug test card 100 back into the terminal insertion slot 7.
③ The sample to be tested carried in the fiber bundle 201 enters the test chamber 103 through the inclined channel 107 and reacts with the test paper 3 inside the test chamber 103.
④ The information acquisition unit 4 captures visual effect change information of the test paper 3 through the observation window 1011 and transmits captured data to the data processing unit 5, and the data processing unit 5 processes the data.
   The data processing unit 5 is internally provided with a drug abuse model. The drug abuse model is used for determining, according to the visual effect change of the test paper 3, whether the drug content of the sample to be tested exceeds critical value. Before the test paper 3 fully reacts, the data processing unit 5 obtains an actually measured visual effect change of the test paper 3; then preliminarily determines, in combination with the actually measured visual effect change and the drug abuse model, whether the drug content of the sample to be tested exceeds the critical value; and finally, displays the test result in the form of sounds, pictures, characters, or light change, through the terminal side output device 6, and the supervisor can know whether the testee has taken drugs by checking the terminal side output device 6.
⑤ The testees who are tested by the one-minute rapid drug test device as having taken drugs need to be taken to the base camp for retesting.
⑥ The retest personnel input the identity information of the testee through the information inputting module 10, and then put the blood, urine, or hairs of the testee into the precision test device 13 for retesting. If the retest result of the precision test device 13 shows that the testee has taken drugs, it is deemed that the testee has taken drugs. If the retest result of the precision test device 13 shows that the testee has not taken drugs, it is deemed that the testee has not taken drugs.
⑦ The data search module 12 searches for a past drug abuse records of the testee according to the identity information of the testee. If it is found that the testee has a history of drug abuse, the retest personnel should pay close attention to the testee. The update module 15 obtains the retest result of the precision test device 13 from the second memory 14, and updates the first memory 11 with the reset result. Optionally, the second memory 14 can also store the drug abuse record stored in the one-minute rapid drug test device, and the update module 15 updates the first memory 11 with the drug abuse record stored in the one-minute rapid drug test device.

For the plug-in drug test card 100 provided in this embodiment, the fiber bundle 201 is used to directly extract the sample to be tested, and then the sample to be tested is delivered into the test chamber 103 to react with the test paper 3, which greatly improves the speed of obtaining the sample to be tested, effectively shortens the test time, and lowers the difficulty of the general test work.

Optionally, the fiber bundle 201 is prepared from a hydrophilic material, which not only has good liquid absorption performance, but also can generate a bending deformation under a pressure, thereby pushing out the extracted sample to be tested.

In this embodiment, the material of the fiber bundle 201 includes ultrafine natural fibers and/or ultrafine artificial synthetic fibers. For example, the ultrafine natural fibers can be animal fibers (spider silks, silks, animal down), plant fibers (cotton, etc.), and the like. The ultrafine synthetic fibers can be hydrophilic polyester, hydrophilic polyamide, hydrophilic polyacrylonitrile, hydrophilic polypropylene, hydrophilic polyvinyl alcohol, nanocellulose, glass fibers, and the like.

Further, the single filament fineness of the ultrafine fibers ranges from 0.3 dtex to 1.0 dtex.

Experiments have shown that the fiber bundle 201 provided in this embodiment has sampling time within 10 s, which is significantly shortened compared to the original sampling time of 3 min. Moreover, the fiber bundle 201 provided in this embodiment can be pluggable, making sampling convenient and requiring a small number of samples.

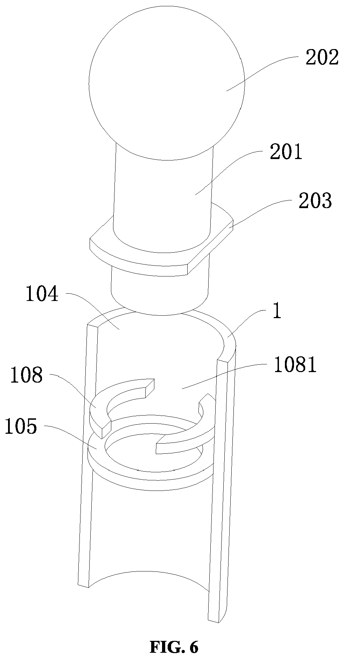

Referring to FIG. 6, in this embodiment, a first limiting boss 105 and a second limiting boss 108 are fixedly arranged in the shell insertion slot 104. The second limiting boss 108 is located on one side of the first limiting boss 105 away from the test paper, and the second limiting boss 108 is provided with a plate passing notch 1081.

A clamping plate 203 that can enter a limiting gap between the first limiting boss 105 and the second limiting boss 108 through the plate passing notch 1081 is arranged outside the sampler 2. When the fiber bundle 201 is inserted into the shell insertion slot 104, the clamping plate 203 is aligned with the plate passing notch 1081 to allow the clamping plate 203 to enter the limiting gap, and the sampler is rotated 90°, that is, the clamping plate 203 is staggered from the plate passing notch 1081 to limit the clamping plate in the limiting gap.

In this case, on the one hand, the first limiting boss 105 stops the clamping plate 203 to limit the sampler 2 from being continued to be inserted and to avoid excessive pushing against the fiber bundle 201. On the other hand, the second limiting boss 108 stops the clamping plate 203 from moving outwards, so that the fiber bundle 201 is in a pushed state all the time, which is conductive to making the sample to be tested be separated from the fiber bundle 201, enter the shell insertion slot 104, and enter the test chamber 103 through the inclined channel 107. The inclined channel 107 that slantways extends to communicate the test chamber 103 with the shell insertion slot 104 is arranged at the other end of the slot bottom of the shell insertion slot 104. Further, the clamping plate 203 also plays a certain role in blocking the flow, which avoids the sample to be tested inside the shell insertion slot 104 from flowing out.

Referring to FIG. 5, two drainage baffle plates 1022 spaced apart from each other is fixedly arranged on one side of the lower shell 102 close to the observation window 1011. The two drainage baffle plates 1022 and an inner wall of the lower shell 102 are encircled to form a test paper slot 1021 with an opening facing the observation window 1011.

One end of the test paper 3 is located in the test paper slot 1021, and the other end extends through the inclined channel 107 into the shell insertion slot 104, thereby providing drainage for the sample to be tested in the shell insertion slot 104, so that the sample to be tested flows along the test paper 3 from the shell insertion slot 104 to the test chamber 103. That is, for the plug-in drug test card 100 provided in this embodiment, the sample to be tested inside is not driven by relying entirely on the gravity, so that the supervisor can achieve the drug abuse test in all postures that the superior holds the shell 1.

Optionally, the angle between the inclined channel 107 and a lengthwise direction of the plug-in drug test card 100 is between 10° and 90°.

In this embodiment, after the clamping plate 203 is inserted into the limiting gap, a buffer space 109 is reserved between the fiber bundle 201 and the inclined channel 107.

It should be noted that the buffer space 109 and the inclined channel 107 can serve as buffer regions for the sample to be tested before the sample to be tested enters the test chamber 103 to buffer the sample to be tested in the shell insertion slot 104, which avoids the problem of insufficient reaction (liquid flushing) of the test paper 3 caused by the rapid influx of a large number of samples into the test chamber 103 after the sampler 2 is pressed.

It is worth pointing out that by the arrangement of the test paper slot 1021, the sample to be tested in the test chamber 103 can be guided to mainly flow in the test paper slot 1021, which avoids ineffective diffusion of the sample to be tested in the test chamber 103, thereby improving the concentration of the sample to be tested. This will accelerate the reaction between the sample to be tested and the test paper 3, thus further shortening the test time.

Optionally, the width of the test paper 3 is 0.5 mm to 2.5 mm. Reducing the width of the test paper 3 appropriately can also shorten the reaction time.

Of course, in some other embodiments, several supporting bosses can also be arranged on an inner side of the lower shell 102. After the test paper 3 is placed on each supporting boss, the upper shell 101 is buckled to clamp and fix the test paper 3.

The rapid drug test method and the distributed drug test system provided in this embodiment have the following advantages:
① When the fiber bundle 201 with the ultrafine fibers is used for sampling, a sufficient sample to be tested can be collected within 10 s, which greatly shortens the sampling time.
② The test paper 3 is of a narrow and elongated structure, which effectively reduces a desired dosage of the sample to be tested. Furthermore, the test paper reacts rapidly and can complete the reaction within 30 s to 40 s and obtain the test result.
③ The test paper 3 is placed in the test paper slot 1021 to accelerate the flowing speed of the sample to be tested under the capillary action, thus effectively shortening the reaction time.
④ The test paper 3 undergoes an elastic deformation in the inclined channel 107, that is, the test paper 3 also forms a certain inclined angle with the lengthwise direction of the plug-in drug test card 100, thereby enlarging a contact area between the test paper 3 and the sample to be tested. Within unit time, the amount of the sample to be tested that is in contact with the test paper 3 increases, thereby shortening the reaction time of the test paper 3.
⑤ The distance between an end surface of the first limiting boss 105 that resists against the clamping plate 203 and an end surface of the extrusion boss 106 that extrudes the fiber bundle 201 is less than a length size of the fiber bundle 201 in a natural state. Therefore, after the sampler 2 is inserted into the shell insertion slot 104, the fiber bundle 201 undergoes a compression bending deformation, thereby extruding the sample to be tested contained in the fiber bundle. Moreover, the sample to be tested will move directionally under the action of a pressure. Therefore, the test paper 3 does not need to be strictly placed horizontally like the existing test paper during operation. The test paper 3 can adapt to more application scenarios.
⑥ The clamping plate 203 is in interference fit with the shell insertion slot 104. After the sampler 2 is inserted into the shell insertion slot 104, the clamping plate 203 can be clamped tightly in the shell insertion slot 104 to prevent the sampler 2 from being separated from the shell 1 in the test process.
⑦ The inclined channel 107 can play a buffering role to prevent the sample to be tested from quickly entering the test chamber 103 and improve the accuracy of the test result.
⑧ The use of the portable terminal 200 for automatic determination reduces the reliance on manpower, which improves the accuracy of the test result and lowers the risk of misjudgment.
⑨ The one-minute rapid drug test device is used for general test, which shortens the test time and improves the test efficiency. The central device 300 is used for retest, which lowers the risk of misjudgment, achieving a better balance between the efficiency and the accuracy, and better overall performance.
⑩ Providing the supervisor with the past drug abuse records of the testees is beneficial for supervisors to prepare mentally and strengthen prevention in advance.
⑪ The preliminary test increases the speed, and the retest lowers the risk of misjudgment. The reaction is sensitive, and the false positive rate is small.
⑫ During the rapid enhancement period of the fluorescence intensity of the test paper 3, the fluorescence intensities of the sample to be tested are continuously collected, and the fluorescence intensity change rates at the corresponding time points are calculated. The fluorescence intensity serving as the x-coordinate and the fluorescence intensity change rate serving as the y-coordinate are fitted to obtain the actually measured curve 19. Then, by the comparison with the drug abuse model, a qualitative test result is output, so that it is not necessary to test, analyze, and compare the fluorescence intensities after the full reaction of the test paper 3, thus achieving rapid outputting of a result and shortening the test time.
⑬ The sampling time is shorter than 10 s, and the visual effect change determining time of the test paper 3 is less than 50 s, so the comprehensive test time is shorter than 1 min.
⑭ The tester paper 3 uses fluorescent nanoparticles doped with rare earth, which improves the sensitivity and can display the test result quickly.
⑮ The one-minute rapid drug test device is small in volume and light in weight, can be designed as a handheld terminal, is convenient to carry, has good concealment and flexibility, and can facilitate the supervisors to hide their identities before approaching the testees, thus preventing the testees from escaping. Secondly, the device is convenient to carry, so that the supervisors use the device flexibly in various scenarios.

It should be understood that although this specification is described according to the implementations, not each implementation only includes one independent technical solution. This narration method of this specification is only for clarity. A person skilled in the art should regard this specification as a whole, and the technical solutions in the respective implementations can also be appropriately combined to form other implementations that can be understood by a person skilled in the art.

A series of detailed explanations listed above are only specific explanations for feasible implementations of the present disclosure, and are not intended to limit the protection scope of the present disclosure. Any equivalent implementations or changes made without departing from the spirit of the present disclosure shall all fall within the protection scope of the present disclosure.

## Claims

1. A rapid drug test method, including: providing drug abuse model, wherein the drug abuse model is used for determining, according to the visual effect change of test paper, whether the drug content of a sample to be tested exceeds a critical value; enabling the sample to be tested to react with the test paper; before the test paper fully reacts, obtaining the actually measured visual effect change of the test paper; and preliminarily determining, in combination with the actually measured visual effect change and the drug abuse model, whether the drug content of the sample to be tested exceeds the critical value, and obtaining preliminary determination result.

2. The rapid drug test method according to claim 1, wherein the determining, according to visual effect change of test paper, whether the drug content of a sample to be tested exceeds critical value comprises: preparing a calibration sample with its drug content equal to the critical value; enabling the calibration sample to react with calibration test paper, obtaining visual effect intensities of the calibration test paper at several moments in the whole reaction process, and calculating visual effect intensity change rates corresponding to the various visual effect intensities; and drawing a drug abuse critical curve that represents the mapping relationship between the visual effect intensity change rates and the visual effect intensities; and
the obtaining actually measured visual effect change of the test paper comprises: obtaining visual effect intensities of the test paper at several moments within a specified time period before the sample to be tested fully reacts with the test paper, and calculating visual effect intensity change rates corresponding to the various visual effect intensities; and drawing an actually measured curve that represents a mapping relationship between the visual effect intensity change rates of the test paper and the visual effect intensities.

3. The rapid drug test method according to claim 2, wherein the preliminarily determining whether the drug content of the sample to be tested exceeds the critical value specifically comprises: preliminarily determining, according to the relative position relationship between the drug abuse critical curve and the actually measured curve, whether the drug content of the sample to be tested exceeds the critical value.

4. The rapid drug test method according to any one of claims 1 to 3, wherein after the preliminary determination result is obtained, the method further comprises: carrying out retest with higher test precision on the testees, who are preliminarily determined that the drug content of the sample to be tested exceeds the critical value, and obtaining retest result.

5. A one-minute rapid drug test device, comprising: plug-in drug test card, configured to enable sample to be tested to react with test paper; and a portable terminal, configured to: provide drug abuse model, wherein the drug abuse model is used for determining, according to the visual effect change of the test paper, whether the drug content of the sample to be tested exceeds critical value; before the test paper fully reacts, obtain an actually measured visual effect change of the test paper; and preliminarily determine, in combination with the actually measured visual effect change and the drug abuse model, whether the drug content of the sample to be tested exceeds the critical value, wherein the plug-in drug test card is detachably connected to the portable terminal.

6. The one-minute rapid drug test device according to claim 5, wherein the plug-in drug test card comprises a sampler configured to obtain the sample to be tested, a shell connected to the sampler in a plugging manner, and the test paper located in the shell.

7. The one-minute rapid drug test device according to claim 6, wherein a test chamber for storing the test paper is arranged inside the shell; a shell insertion slot communicated with the test chamber is arranged on the end surface of one end of the shell; the sampler comprises a fiber bundle connected to the shell insertion slot in a plugging manner and a handheld part fixed at one end of the fiber bundle away from the shell.

8. The one-minute rapid drug test device according to claim 7, wherein an inclined channel that slantways extends to communicate the test chamber with the shell insertion slot is arranged at the slot bottom of the shell insertion slot; and one end of the test paper extends into the shell insertion slot through the inclined channel.

9. The one-minute rapid drug test device according to claim 8, wherein a first limiting boss is fixedly arranged in the shell insertion slot; a clamping plate is arranged outside the sampler; and when the sampler is inserted into the shell insertion slot, the first limiting boss stops the clamping plate to limit the sampler from being continued to be inserted.

10. The one-minute rapid drug test device according to claim 9, wherein a second limiting boss is arranged on one side of the first limiting boss away from the test paper; the second limiting boss is provided with plate passing notch; and the clamping plate that enters a limiting gap between the first limiting boss and the second limiting boss through the plate passing notch is arranged outside the sampler.

11. The one-minute rapid drug test device according to claim 10, wherein after the clamping plate is clamped into the limiting gap, a buffer space is reserved between the fiber bundle and the inclined channel.

12. The one-minute rapid drug test device according to any one of claims 6 to 11, wherein
terminal insertion slot is arranged on one surface of the portable terminal, and the plug-in drug test card is connected to the terminal insertion slot in a plugging manner;
the shell is provided with observation window configured to observe the visual effect change of the test paper;
information acquisition unit configured to obtain the visual effect change is arranged at a position, corresponding to the observation window, in the terminal insertion slot;
the portable terminal is further provided with: data processing unit, wherein the data processing unit is connected to the information acquisition unit and is configured to process data acquired by the information acquisition unit and generate test result; and terminal side output device, wherein the terminal side output device is electrically connected to the data processing unit and is configured to provide displaying of at least one of pictures, characters, videos, sounds, or light for a supervisor according to the test result.

13. A distributed drug test system, comprising a central device and the one-minute rapid drug test device according to any one of claims 5 to 12, wherein the central device is provided with a precision test device; the one-minute rapid drug test device and the precision test device are both configured to conduct drug abuse test; the test speed of the one-minute rapid drug test device is higher than the test speed of the precision test device; and the test precision of the precision test device is higher than the test precision of the one-minute rapid drug test device.

14. The distributed drug test system according to claim 13, wherein the central device further comprises:
information inputting module, configured to obtain identity information of testees;
first memory, configured to store all drug abuse records that have been checked by the supervision department; and
data search module, wherein the data search module is electrically connected to the information inputting module and the first memory respectively and is configured to call, according to the identity information, drug abuse records related to the testees.

15. The distributed drug test system according to claim 13 or 14, wherein the precision test device comprises at least one of a urine test device, a blood test device, a hair test device, a sweat test device, a saliva test device, or a kidney function test device.
